# EUROPEAN PATENT APPLICATION

(11) **EP 2 481 411 A1**
(43) Date of publication of application: **01.08.2012**
(21) Application number: 11000677.2
(22) Date of filing: 27.01.2011
(51) Int. Cl.: A61K 31/515, A61K 9/20, A61K 9/28, A61K 9/22

(54) **Oral dosage forms for modified release comprising the JAK3 inhibitor tasocitinib**

(71) Applicant: Ratiopharm GmbH, 89070 Ulm (DE)
(72) Inventor: Stefan, Ralph, 88370 Ebenweiler (DE); Sievert, Frank, Dr., 89604 Allmendingen (DE)
(74) Representative: Aechter, Bernd

(57) **Abstract**

The invention essentially relates to oral dosage forms comprising a JAK3 inhibitor, preferably tasocitinib, suitable for modified release, and processes of preparing such oral dosage forms.

## Description

### Background

The invention essentially relates to oral dosage forms comprising a pharmaceutically active substance, preferably 3-{(3R,4R)-4-methyl-3-[methyl-(7H-pyrrolo[2,3-d]pyrimidin-4-yl)-amino]-piperidin-1-yl}3-oxo-propionitrile, suitable for modified release, and processes of preparing such oral dosage forms.

3-{(3R,4R)-4-methyl-3-[methyl-(7H-pyrrolo[2,3-d]pyrimidin-4-yl)-amino]-piperidin-1-yl}3-oxo-propionitrile apparently has the chemical formula C₁₆H₂₀N₆O and is reported in WO 03/048126 as an inhibitor of protein kinases, such as the enzyme Janus Kinase 3 (hereinafter also referred to as "JAK3") and as such it has been asserted that it is useful in therapy as immunosuppressive agents for organ transplants, xeno transplantation, lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia and other indications, where immunosuppression would be desirable (see WO 03/048126), and is known under the INN tasocitinib. The 3-{(3R,4R)-4-methyl-3-[methyl-(7H-pyrrolo[2,3-d]pyrimidin-4-yl)-amino]-piperidin-1-yl}3-oxo-propionitrile apparently has the chemical structure of formula (I):

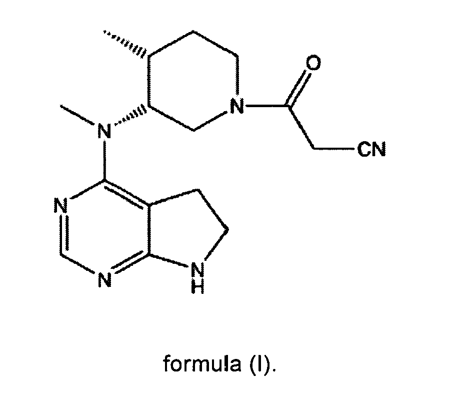

In this regard it is noted that the compound according to formula (I) would seem to refer to 3-{(3R,4R)-4-methyl-3-[methyl-(7H-pyrrolo[2,3-d]pyrimidin-4-yl)-amino]-piperidin-1-yl}3-oxo propionitrile (= tasocitinib) or its solvates or hydrates as well as pharmaceutical acceptable salts thereof are said to be obtained according to the procedures as outlined in WO 02/096909. The mono citrate form has apparently been described in WO 03/048162.

Whereas the prior art (WO 03/048162, WO 02/096909) mentions that tasocitinib might be formulated into pharmaceutical compositions, no specific formulations have been disclosed.

It was reported that tasocitinib is difficult to formulate. The solubility of the drug apparently is undesirably low. Various physiological factors such as gastrointestinal pH, enzyme activities, gastric and intestinal transit rates negatively influenced the bioavailability of tasocitinib. In addition it was reported that especially low dose tasocitinib formulations generally suffered from the difficulty of providing a sufficient content uniformity.

Hence, there is a need for the provision of pharmaceutical dosage forms and processes for the manufacture of these pharmaceutical dosage forms comprising tasocitinib, which do not suffer from the above mentioned draw-backs. Preferably, an oral dosage form should be provided having improved properties like content-uniformity, solubility, dissolution profile, well-defined, predictable and reproducible dissolution rates, stability and bioavailability. Such an oral dosage form should be producible in a large scale in an economic beneficial way.

### Summary of the Invention

The present invention provides an oral dosage form for modified release that can overcome the above drawbacks, the oral dosage form for modified release comprising
(a) tasocitinib, and
(b) a non-erodible material.

It was found that the dosage forms of the present invention have the advantage that the tasocitinib is gradually released over a relatively long period so that the drug is maintained in the blood stream for a long time and at a uniform concentration. This allows administration, e.g., only once daily. Administration of the oral dosage forms of the present invention result in little blood level fluctuation, that means periods of transient therapeutic overdose, followed by a period of therapeutic underdosing can be avoided. Consequently, the dosage forms of the present invention, particularly provide a constant release of tasocitinib, preferably over a prolonged period of time, which avoids blood level fluctuations of the drug in the patient.

Moreover, the dosage form of the present invention is released in the gastrointestinal tract of the patient but not in the stomach, in order to avoid a "nervous stomach" or nausea.

A further subject of the present invention is a process for manufacturing the oral dosage forms of the present invention, preferably in form of a modified release tablet.

### Detailed Description of the Invention

In the following, explanations regarding the pharmaceutical dosage form of the present invention are given. However, these explanations also apply to the processes for manufacturing the pharmaceutical dosage form, such as the modified release tablet of the present invention, and to the use of the present invention.

Within the present application generally the term "modified release" is used as defined by the USP. Preferably, modified release dosage forms are those whose drug release characteristics accomplish therapeutic or convenience objectives not offered by immediate release forms. Generally, immediate release (IR) forms release at least 70 % of the drug within 1 hour or less. The term "modified release" can comprise delayed release, prolonged release, sustained release, extended release and/or controlled release.

Delayed release usually indicates that the drug (i.e., tasocitinib) is not being released immediately after administration but at a later time, preferably less than 10 % are released within two hours after administration.

Prolonged release usually indicates that the drug (i.e., tasocitinib) is provided for absorption over a longer period of time than IR forms, preferably for about 2 to 24 hours, in particular for 3 to 12 hours.

Sustained release usually indicates an initial release of drug (i.e., tasocitinib), sufficient to provide a therapeutic dose soon after administration, preferably within two hours after administration, and then a gradual release after an extended period of time, preferably for about 3 to 18 hours, in particular for 4 to 8 hours.

Extended release usually indicates a slow drug (i.e., tasocitinib) release, so that plasma concentrations are maintained at a therapeutic level for a time period of between 6 and 36 hours, preferably between 8 and 24 hours.

Controlled release dosage forms usually release the drug (i.e., tasocitinib) at a constant rate and provide plasma concentrations that remain essentially invariant with time.

In a preferred embodiment, the oral dosage form of the present invention is an extended release dosage form.

In particular, the oral dosage form of the present invention shows a drug release of less than 10 % within 2.0 hours. Further, the oral dosage form of the present invention shows a drug release of more than 80 % within 3.0 to 12.0 hours, preferably between 4.0 and 8.0 hours.

Generally, within this application the release profile is determined according to USP 31-NF26 release method, apparatus II (paddle). The measurements are carried out in 0.1 HCl at 37 °C, wherein the stirring speed was 75 rpm, and re-buffering after 2 hours to pH 6.8.

In a preferred embodiment, the oral dosage form of the present invention is a solid oral dosage form, in particular a solid peroral dosage form.

The term tasocitinib (component (a)) as used in the present invention relates to the compound as shown in formula I (free base) or to its acid form or its basic form. That means, "tasocitinib" as used in the present invention also relates to the pharmaceutically acceptable salts, preferably pharmaceutically acceptable acid addition salts, e.g., as described in WO 02/096909. The acids, which are used to prepare the pharmaceutically acceptable acid addition salts, are preferably those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [1,1'-methylene-bis-(2-hydroxy-3-naphthoate)] salts.

The term "tasocitinib" also relates to stereospecific base addition salts of formula (I). The chemical bases that may be used as reagents to prepare pharmaceutically acceptable base salts of those compounds of formula I that are acidic in nature are those that form non-toxic base salts with such compounds. Such non-toxic base salts include, but are not limited to, those derived from such pharmacologically acceptable cations, such as alkali metal cations (e.g., potassium and sodium) and alkaline earth metal cations (e.g., calcium and magnesium), ammonium or water soluble amine addition salts, such as N-methylglucamine-(meglumine), and the lower alkanol ammonium and other base salts of pharmaceutically acceptable organic amines.

In the oral dosage form of the present invention, tasocitinib as the active ingredient (component (a)) can be provided in amorphous form, in crystalline form or as a mixture of both forms. Preferably, tasocitinib is present in crystalline form, wherein the crystalline modification is as described in WO 03/048162. In a particularly preferred embodiment of the present invention tasocitinib is provided as the citrate or hemi citrate. Most preferred is the crystalline form of the citrate or hemi citrate of tasocitinib.

In a preferred embodiment, the oral dosage form of the present invention comprises 1.0 to 60 wt.%, more preferably 2.0 to 30 wt.-%, still more preferably 3.0 to 20 wt.%, in particular 4.0 to 15 wt.% tasocitinib, based upon the total weight of the oral dosage form and based on the weight of tasocitinib in form of the free base, i.e. as shown in formula (I) above.

In a preferred embodiment, the oral dosage form of the present invention comprises 1.0 to 100 mg, more preferably 2.0 to 50 mg, still more preferably 3.0 to 20 mg, in particular 4.0 to 12 mg tasocitinib, based upon the total weight of the oral dosage form and based on the weight of tasocitinib in form of the free base, i.e. as shown in formula (I) above.

The modified release tablet of the present invention further contains a non-erodible material (b). Generally, the non-erodible material is suitable as release controlling agent.

In a first embodiment, the non-erodible material can be described as providing a scaffold (matrix) for embedding the active ingredient and to form a physical barrier, which hinders the active ingredient from being released immediately from the dosage form. Thus, the non-erodible material has the effect that the active ingredient can be released from the scaffold in continuous manner. Release of the drug from the matrix can further be by dissolution controlled as well as diffusion controlled mechanisms. In this first embodiment the non-erodible material functions as matrix forming material.

In a second embodiment, the non-erodible material can be described as a shell-forming material. Preferably, in that embodiment the oral dosage form is a tablet. The release modifying shell preferably encompasses the drug containing tablet core.

In a third embodiment, the non-erodible material can be described as a release modifying coating in a multiple unit pellet system (MUPS).

Generally, (i.e. for all three above described embodiments) the oral dosage form of the present invention further comprises a non-erodible material (b). Non-erodible materials are materials, which are able to provide modified release properties, preferably due to their limited solubility, more preferably due to their limited solubility in aqueous conditions at pH 5.0. Preferably, the non-erodible polymer has a water solubility of less than 33 mg/l at a temperature of 25°C at a pH of 5.0, more preferably of less than 22 mg/l, still more preferably of less than 11 mg/l, especially from 0.01 to 5 mg/l. The water-solubility is determined according to the column elution method of the Dangerous Substances Directive (67/548/EEC), Annex V, Chapter A6. The pH value is determined according to Ph.Eur. 6.0, 2.2.3. The pH value of the aqueous medium usually is achieved by addition of HCl (or NaOH), if necessary.

The solubility of the non-erodible material can be pH independent or pH dependent. Both embodiments are preferred. If the non-erodible material is pH dependent, it is preferred that the non-erodible material has a solubility in water at 25 °C at a pH of 7.0 of more than 33 g/I, more preferably of 50 g/I to 10,000 g/l, still more preferably from 100 g/l to 5,000 g/l, in particular from 200 g/I to 2,000 g/I.

The non-erodible material can comprise an inert non-erodible material, a lipid non-erodible material and/or a hydrophilic non-erodible material. Examples for an inert non-erodible material are ethylcellulose, methacrylate copolymer, polyamide, polyethylene, and polyvinyl acetate; examples for lipid non-erodible materials are carnauba wax, cetyl alcohol, hydrogenated vegetable oils, microcrystalline waxes, monoglycerides, triglycerides and PEG monostearate; examples for hydrophilic non-erodible materials are alginates, carbopol, gelatin, hydroxypropyl cellulose, hydroxypropyl methylcellulose, methylcellulose, xanthan gum and polyethylene oxide.

In a preferred embodiment, the non-erodible material is a non-erodible polymer. The non-erodible polymer usually has a weight average molecular weight ranging from 30.000 to 3,000,000 g/mol, preferably from more than 50,000 to 2,500,000 g/mol, more preferably from more than 125,000 to 2,000,000 g/mol, still more preferably from 250,000 to 2,200,000 g/mol, particularly preferred from 400,000 to 1,500,000 g/mol. Furthermore, a 2 % w/w solution of the non-erodible polymer in water at pH 7.0 preferably has a viscosity of more than 2 mPas, more preferably of more than 5 mPas, particularly more than 8 mPas and up to 850 mPas, when measured at 25°C. The viscosity is determined according to Ph. Eur., 6^{th} edition, Chapter 2.2.10. In the above definition the term "solution" may also refer to a partial solution (in case that the polymer does not dissolve completely in the solution). The weight average molecular weight is preferably determined by gel electrophoresis.

It is further preferred that the non-erodible polymer has a melting temperature of below 220 °C, more preferably of between 25 °C and 200 °C. In a particularly preferred embodiment the melting temperature is between 35°C and 190°C. The determination of the melting temperature is carried out according to Ph. Eur., 6th edition, Chapter 2.2.15.

If the non-erodible material (b) is a polymeric material, it preferably can be selected from acrylic polymers or acrylic copolymers such as polymers obtained from acrylic acid and/or methacrylic acid monomers. Other preferred polymers include, but are not limited to, cellulose and cellulose derivatives such as cellulose acetate phthalate (CAP), hydroxypropyl methyl cellulose (HPMC), hydroxypropyl methyl cellulose acetate (HPMCA), hydroxypropyl methyl cellulose phthalate (HPMCP) and cellulose acetate succinate (CAS), polyvinyl polymers such as polyvinyl alcohol phthalate, polyvinyl acetate phthalate and polyvinyl butyl phthalate, and mixtures of one or more of these polymers.

In particular, the following kinds of non-erodible polymers are particularly preferred.
1. Cellulose ether, preferably ethyl cellulose, preferably ethyl cellulose having an average molecular weight of 150,000 to 300,000 g/mol and/or an average degree of substitution, ranging from 1.8 to 3.0, preferably from 2.2 to 2.6. This embodiment preferably is used for MUPS or core/shell-tablets;
2. Cellulose ester, preferably cellulose acetate phthalate, carboxymethyl ethyl cellulose, hydroxypropyl methylcellulose phthalate. This embodiment is preferably used for core/shell tablets;
3. Copolymers of methacrylic acid or methacrylic acid esters, preferably ethylacrylate-methylmethacrylate-trimethylammonioethylmethacrylate-chloride ethylacrylate-methylmethacrylate-trimethylammonioethylmethacrylate-chloride ethylacrylate-methylmethacrylate, methacrylic acidmethylmethacrylate, wherein the weight ratio is 1 : 2 methacrylic acid-methylmethacrylate, wherein the weight ratio is 1 : 1;
4. Polyvinyl acetate or polyvinyl acetate copolymers, preferably polyvinyl acetate phthalate; and mixtures thereof.

Preferred acrylic polymers are, for example, polyacrylate, polymethacrylate as well as derivatives and mixtures or copolymers thereof. The polyacrylates used in the invention preferably show the above indicated parameters (e.g. weight average molecular weight, solubility, etc).

In a preferred embodiment the non-erodible acrylic polymer (b) is a polymer consisting of the structures according to the general formulae (2) and (3). 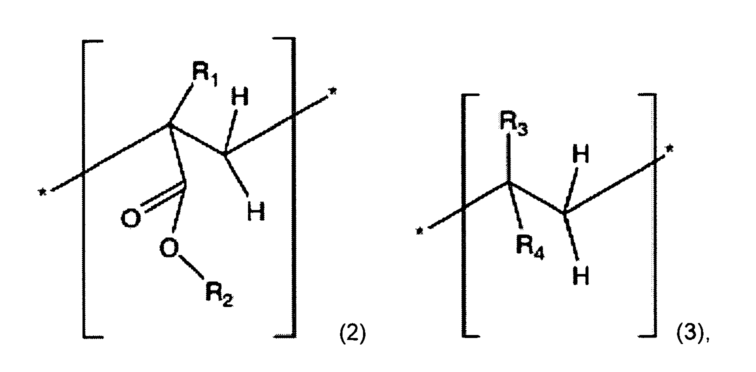 wherein in formulae (2) and (3)
R₁ is a hydrogen atom or an alkyl group, preferably a hydrogen atom or a methyl group or an ethyl group, particularly preferred a methyl group;
R₂ is a hydrogen atom or an alkyl group, preferably a hydrogen atom or a C₁-C₄ alkyl group, particularly preferred a methyl group, ethyl group or butyl;
R₃ is a hydrogen atom or an alkyl group, preferably a hydrogen atom or a methyl group;
R₄ is an organic group, preferably a carboxylic acid or a derivative thereof, particularly preferred a group according to the formula -COOH, -COOR₅,
R₅ is an alkyl group or a substituted alkyl group, preferably a methyl, ethyl, propyl or butyl group, or -CH₂-CH₂-N(CH₃)₂ or -CH₂-CH₂-N(CH₃)₃⁺ halogen- (in particular Cl⁻) as substituted alkyl group.

The acrylic polymer (b) according to formulae (2) and (3) is usually comprised of structures with a molar ratio of from 1 : 40 to 40 : 1. The preferred ratio of the structures of formula (2) to structures of formula (3) is from 2 : 1 to 1 : 1, particularly 1 : 1. When R₄ is -COO -CH₂-CH₂-N(CH₃)₃⁺Cl⁻, the ratio of structures according to formula (2) to structures of formula (3) preferably is 20 : 1 to 40 : 1.

In case of an alternating copolymerization with a ratio of 1 : 1, this results in a preferred polymer according to formula (2+3)

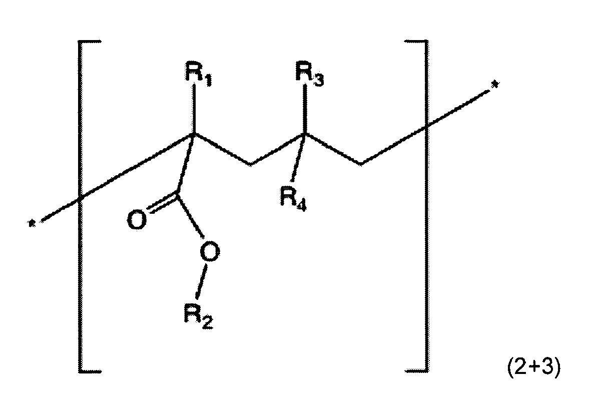

Polyacrylates according to the formulae (2) and (3) as mentioned above are particularly preferred, wherein R₁ and R₃ are alkyl, particularly methyl, R₂ is methyl and/or ethyl and R₄ is hydrogen or -COO-CH₂-CH₂-N(CH₃)₃⁺Cl⁻. A particularly preferred ratio of the structures according to formula (2) to the structures according to formula (3) is 1 : 1 or 1 : 20. A corresponding polymer preferably has a weight average molecular weight of from 50,000 to 250,000 g/mol, more preferred of from 120,000 to 180,000 g/mol.
In a particularly preferred embodiment in formula (2) (or in formula (2+3) as well), as indicated above, R₂ is both a methyl and a butyl group, whereby the ratio methyl to butyl group preferably is 1 : 1.

Further, the acrylic polymer preferably can be a ternary polymer comprising the structures according to the general formulae (2a), (2b) and (3) 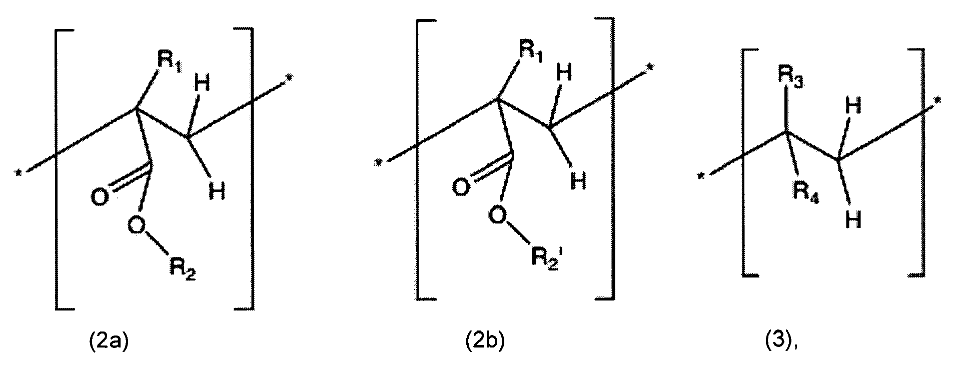 wherein R₁ and R₃ are alkyl, particularly methyl, R₂ is methyl, R_{2'} is ethyl and R₄ is -COO-CH₂-CH₂-N(CH₃)₃⁺Cl⁻.

The non-erodible material (b) is contained in the tablet in an amount of 5 to 80 wt.%, preferably from 10 to 50 wt.%, most preferably from 15 to 40 wt.%, based upon the total weight of the oral dosage form. If too little non-erodible material is used, the formulations may break up during the passage down the gastrointestinal tract and this, in turn, may lead to a premature release of a large portion of the content of the drug. If too much matrix former is used, there is a risk that some of the drug will be encapsulated and not released from the tablet.

The oral dosage form of the invention further optionally comprises a pore-forming material (c). The term "channelling agent" is in the art often synonymously used for the pore-forming material of the present invention. Since the pore-forming material is generally soluble in the gastrointestinal tract and leaches out from the oral dosage form, the pore-forming material can be described has having the effect of forming pores, such as small holes within the tablet, through which the active ingredient can be released from the tablet matrix in a controlled manner. Thus, release of the active ingredient generally depends on dissolving the pore forming material and thereby forming a porous matrix of capillaries such that the drug can leach out of the matrix.

The pore-forming substance usually has a water solubility of more than 50 mg/l, preferably more than 100 mg/l, at a temperature of 25 °C and pH 5.0, more preferred of more than 250 mg/l and particularly preferred of more than 25 g/I. The water-solubility of the pore-forming substance may range up to 2.5 kg/l. The water-solubility is determined according to the column elution method of the Dangerous Substances Directive (67/548/EEC), Annex V, Chapter A6.

The pore-forming substances can be selected from inorganic substances, preferably from inorganic salts such as NaCl, KCI, Na₂SO₄. Furthermore, the pore-forming substances can be selected from organic substances, in particular from organic substances being solid at 30 °C and having the above-mentioned water solubility. Suitable examples are PEG, particularly PEG, having a weight average molecular weight of from 2,000 to 10,000 g/mol.

Furthermore, polyvinylpyrrolidone, preferably having a weight average molecular weight of from 5,000 to 29,000 g/mol, PEG with a weight average molecular weight of 380 - 4800, polyethylene oxide with a weight average molecular weight of less than 100,000 and a viscosity of less than 20 mPa·s, sugar alcohols like mannitol, sorbitol, xylitol, isomalt, and mono or disaccharides, like lactose, are also suitable as pore-forming substances.

The pore forming material is usually contained in the tablet in an amount of 1 to 50 wt.%, preferably from 2 to 40 wt.%, most preferably from 5 to 30 wt.%, based upon the total weight of the oral dosage form.

The tablet of the present invention can further comprise at least one excipient (d) selected from solubilizers (d1), fillers (d2), disintegrants (d3), lubricants (d4), surfactants (d5), glidants (d6), anti-sticking agents (d7), plasticizers (d8) and mixtures thereof.

The composition of the subject invention preferably comprises one or more solubilizers, preferably hydrophilic solubilizers. Generally, the term "solubilizer" means any organic excipient, which is capable of improving the solubility and/or dissolution of the active pharmaceutical ingredient. Generally, the term "hydrophilic solubilizer" means any organic excipient, which possesses hydrophilic groups and is capable of improving the solubility and/or dissolution of the active pharmaceutical ingredient. Preferably, the hydrophilic solubilizer is capable of reducing the dissolution time of a pharmaceutical composition by 5 %, more preferably by 20 %, according to USP 31-NF26 release method, using apparatus 2 (paddle), compared to the same pharmaceutical composition comprising calcium hydrogen phosphate instead of the hydrophilic solubilizer.

The solubilizers are selected, for example, from the group of known inorganic or organic excipients. Such excipients preferably include polymers, low molecular weight oligomers and natural products.

Preferably, the hydrophilic solubilizer is a water-soluble compound, having a water solubility of more than 10 mg/l, more preferably of more than 20 mg/l, still more preferably of more than 50 mg/l at a temperature of 25 °C. The solubility of the hydrophilic solubilizer might be e.g. up to 1,000 mg/l or up to 300 mg/ml at a temperature of 25 °C. The water-solubility is determined according to the column elution method of the Dangerous Substances Directive (67/548/EEC), Annex V, Chapter A6.

In a preferred embodiment the solubilizer is a hydrophilic polymer, preferably having the above-mentioned water-solubility. Generally, the term "hydrophilic polymer" encompasses polymers comprising polar groups. Examples for polar groups are hydroxy, amino, amido, carboxy, carbonyl, ether, ester and sulfonate. Amido groups are particularly preferred.

The hydrophilic polymer usually has a weight average molecular weight, ranging from 1,000 to 250,000 g/mol, preferably from 2,000 to 100,000 g/mol, particularly from 4,000 to 75,000 g/mol. Furthermore, a 2 % w/w solution of the hydrophilic polymer in pure water preferably has a viscosity of from 1 to 20 mPa·s, more preferably from 2 to 8 mPa·s at 25 °C. The viscosity is determined according to the European Pharmacopoeia (hereinafter referred to as Ph. Eur.), 6^{th} edition, Chapter 2.2.10.

Furthermore, the hydrophilic polymer used as hydrophilic solubilizer preferably has a glass transition temperature (T_{g}) or a melting point of 25 °C to 200 °C, more preferably of 90 °C to 170 °C. The glass transition temperature, T_{g}, is the temperature, at which the hydrophilic polymer becomes brittle on cooling and soft on heating. That means, above Tg, the hydrophilic polymers become soft and capable of plastic deformation without fracture. The glass transition temperature or the melting point are determined with a Mettler-Toledo^{®} DSC 1, wherein a heating rate of 10 °C per minute and a cooling rate of 15 °C per minute is applied. The determination method essentially is based on Ph. Eur. 6.1, section 2.2.34. For the determination of T_{g}, the polymer is heated twice (i.e. heated, cooled, heated).

More preferably, derivatives of cellulose (e.g. hydroxyproply methyl cellulose (HPMC), preferably having a weight average molecular weight from 20,000 to 90,000 g/mol, and/or preferably a ratio of methyl groups from 10 to 35 %, and preferably a ratio of hydroxypropyl groups from 1 to 35 %; hydroxypropyl cellulose (HPC), preferably having a weight average molecular weight of from 40,000 to 100,000 g/mol), polyvinylpyrrolidone, preferably having a weight average molecular weight of from 10,000 to 60,000 g/mol, copolymers of polyvinylpyrrolidones, preferably copolymers comprising vinylpyrrolidone and vinylacetate units (e.g. Povidon^{®} VA 64; BASF), preferably having a weight average molecular weight of 40,000 to 75,000 g/mol, polyoxyethylene alkyl ethers, co-blockpolymers of ethylene oxide and propylene oxide, preferably having a polyethylene content of 70 to 90 wt.% and/or preferably having a weight average molecular weight from 1,000 to 50,000 g/mol, in particular from 3,000 to 25,000 g/mol, polyvinyl alcohol, polyethylene glycol, preferably having a weight average molecular weight ranging from 1,000 to 50,000 g/mol, are used as hydrophilic solubilizers. The weight average molecular weight is preferably determined by gel electrophoresis.

In particular, polyvinylpyrrolidone and copolymers of polyvinylpyrrolidone, in particular copolymers comprising vinylpyrrolidone and vinylacetate units, having the structure 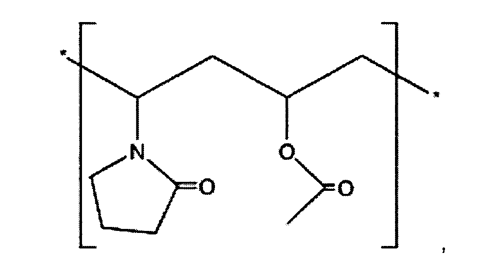 can be used as hydrophilic solubilizers.

It is particularly preferred that the above-mentioned kinds of hydrophilic polymers fulfill the functional requirements (molecular weight, viscosity, T_{g}, melting point, non-semipermeable properties), as illustrated above.

In the pharmaceutical composition of the present invention, at least one of the above-mentioned hydrophilic solubilizers is present. Alternatively, a combination of two or more hydrophilic solubilizers can be employed.

Usually, solubilizers can be used in an amount of 0.1 to 20 wt.%, preferably of 1 to 15 wt.% based on the total weight of the oral dosage form.

Generally, fillers are used to top up the volume for an appropriate oral deliverable dose, when low concentrations of the active pharmaceutical ingredients (about 30 wt.% or lower) are present. Preferred fillers of the invention are calcium phosphate, saccharose, calcium carbonate, calcium silicate, magnesium carbonate, magnesium oxide, maltodextrin, glucopyranosyl mannitol, calcium sulfate, dextrate, dextrin, dextrose, hydrogenated vegetable oil and/or cellulose derivatives, such as microcrystalline cellulose. A pharmaceutical composition according to the invention may comprise an inorganic salt as a filler. Preferably, this inorganic salt is dicalcium phosphate, preferably in form of the dihydrate (dicafos).

Dicalcium phosphate dihydrate is insoluble in water, non-hygroscopic, but still hydrophilic. Surprisingly, this behavior contributes to a high storage stability of the composition.

Usually, fillers can be used in an amount of 0 to 60 wt.%, preferably of 5 to 40 wt.%, based on the total weight of the composition.
The oral composition of the present invention can further comprise one or more of a disintegrant. In a preferred embodiment of the invention, the tablet does not contain a disintegrant.

Generally, disintegrants are compounds, capable of promoting the break up of a solid composition into smaller pieces when the composition gets in contact with a liquid, preferably water.

Preferred disintegrants are sodium carboxymethyl starch, cross-linked polyvinylpyrrolidone (crospovidone), sodium carboxymethyl glycolate (e.g. Explotab^{®}), swelling polysaccharide, e.g. soya polysaccharide, carrageenan, agar, pectin, starch and derivates thereof, protein, e.g. formaldehyde - casein, sodium bicarbonate or mixtures thereof. Crospovidone is particularly preferred as disintegrant. Furthermore, a combination of crospovidone and agar is particularly preferred.

Usually, disintegrants can be used in an amount of 0 to 20 wt.%, preferably of 1 to 10 wt.%, based on the total weight of the composition.

In a preferred embodiment of the present invention the oral dosage form is free of any disintegrants.

The oral dosage form of the present invention might further comprise one or more of a surfactant (d4). Preferably, sodium lauryl sulfate is used as surfactant.

Usually, surfactants can be used in an amount of 0.05 to 2 wt.%, preferably of 0.1 to 1.5 wt.%, based on the total weight of the oral dosage form.

Additionally, the oral dosage form of the present invention may comprise a lubricant (d5), a glidant (d6) and/or an anti-sticking agent (d7).

In a preferred embodiment of this invention, a lubricant may be used. Lubricants are generally employed to reduce dynamic friction. The lubricant preferably is a stearate, talcum powder or fatty acid, more preferably, hexanedioic acid or an earth alkali metal stearate, such as magnesium stearate. The lubricant is suitably present in an amount of 0.1 to 3 wt.%, preferably about 0.5 to 1.5 wt.% of the total weight of the composition. Preferably, the lubricant is applied in a final lubrication step during the powder preparation. The lubricant generally increases the powder flowability.

The glidant can for example be colloidal silicone dioxide (e.g. Aerosil^{®}. Preferably, the glidant agent is present in an amount of 0 to 8 wt.%, more preferably at 0.1 to 3 wt.% of the total weight of the composition. Preferably, the silicone dioxide has a specific surface area of 50 to 400 m²/g, measured by gas adsorption according to Ph. Eur., 6th edition, Chapter 2.9.26. multipoint method, volumetric determination

The anti-sticking agent is for example talcum and may be present in amounts of 0.05 to 5 wt.%, more preferably in an amount of 0.5 to 3 wt.% of the total weight of the composition.

Furthermore, in a preferred embodiment the pharmaceutical composition of the present invention further comprises one or more plasticizers (d8). The "plasticizers" usually are compounds capable of lowering the glass transition temperature (Tg) of the non-erodible material, preferably the non-erodible polymer, preferably of lowering T_{g} from 1 to 50 °C, especially from 5 to 30 °C. Plasticizers (d8) usually are low molecular weight compounds (having a molecular weight from 50 to 500 g/mol) and comprise at least one hydrophilic group.

Examples of suitable plasticizers are dibutyl sebacetate (DBS), Myvacet^{®} (acetylated monoglycerides), triacetin (GTA), citric acid esters, like acetyltriethyl citrate (ATEC) or triethyl citrate (TEC), propylene glycol, dibutyl phathalate, diethyl phathalate, or mixtures thereof.

The combined use of the non-erodible polymer (b) and the pore-forming substance (c) and optionally the plasticizer (d8) preferably is capable of modifying the drug release rate. The use of plasticizers is particularly preferred in the third embodiment concerning MUPS.

Regarding the above mentioned pharmaceutically acceptable excipients, the application generally refers to "Lexikon der Hilfsstoffe für Pharmazie, Kosmetik und angrenzende Gebiete", edited by H. P. Fiedler, 5th Edition, Editio Cantor Verlag, Aulendorf and earlier editions, and "Handbook of Pharmaceutical Excipients", third edition, edited by Arthur H. Kibbe, American Pharmaceutical Association, Washington, USA, and Pharmaceutical Press, London.

In the tablet according to the present invention the non-erodible material (b), the pore forming material (c) and/or the at least one excipient (d) preferably have a surface of 0.2 to 10 m²/g, preferably of 0.3 to 8 m²/g, most preferably of 0.4 to 5 m²/g, as measured by gas adsorption according to Ph. Eur., 6th edition, Chapter 2.9.26, multipoint method, volumetric determination.

In the tablet of the invention the at least one non-erodible material (b), the pore forming material (c) and/or the excipient(s) generally show a plastic behavior, such as a ductile behaviour. This behavior can be described by the yield pressure of the material. The materials of components (a), (b) and/or (c) generally have a yield pressure of less than 150 MPa, preferably less then 100 MPa, most preferably of less than 75 MPa. If the yield pressure is above 150 MPa, the material is too brittle and causes difficulties in being compressed into a tablet, bearing the risk that the tablet breaks or crumbles. The yield pressure can be determined from a Heckel plot. According to Heckel, there is a linear relationship between the relative porosity (inverse density) of a powder and the applied pressure. The slope of the linear regression is the Heckel constant, a material dependent parameter inversely proportional to the mean yield pressure (the minimum pressure required to cause deformation of the material undergoing compression). Thus, the yield pressure is obtained by measuring the reciprocal value from the slope of the Heckel plot.

In this context it is generally noted that, due to the nature of pharmaceutical excipients, it cannot be excluded that a certain compound meets the functional requirements of more than one of the above mentioned excipient classes. However, in order to enable an unambiguous distinction and terminology in the present application, the same pharmaceutical compound can only be subsumed as one of the functional excipient classes presented above. For example, if microcrystalline cellulose is used as a filler, it cannot additionally classify as a disintegrant (although microcrystalline cellulose has some disintegrating properties).

As explained above, the present invention concerns three preferred embodiments of the solid oral dosage form. Hence, the present invention further relates to three preferred embodiments of a process for producing said oral dosage forms.
In the first preferred embodiment the present invention concerns a matrix dosage form, preferably a matrix tablet. The matrix tablet preferably is produced by a process, comprising the steps of
(1-I) providing (and optionally blending) components (a), (b), optionally c), and optionally (d),
(1-II) optionally agglomerating the components of step (I) to yield granules,
(1-III) compressing the mixture resulting from step (I) or (II) into tablets; and
(1-IV) optionally coating the tablets, preferably with a suitable film (e).

In this first preferred embodiment of the invention, the dosage form preferably comprises tasocitinib, a non-erodible material, a pore-forming material, a filler, a glidant and a lubricant. In a further preferred embodiment, the composition comprises from 5 to 20 wt.% of tasocitinib, from 25 to 60 wt.% of non-erodible material, from 10 to 40 wt.% of a pore-forming material, from 10 to 40 wt.% of a filler, from 1 to 10 wt.% of a glidant and from 1 to 10 wt.% of a lubricant, based upon the total weight of the dosage form.

In a second preferred embodiment of the invention, the oral dosage form is in form of a tablet, comprising a core and a shell, wherein the core comprises components (a) and optionally (c) and/or (d), and wherein the shell comprises components (b) and optionally (c) and/or (d).

The tablet of the invention preferably is produced by a process, comprising the steps of
(2-I) mixing components (a) and optionally (c) and/or (d),
(2-II) optionally agglomerating the components of step (I) to yield granules,
(2-III) compressing the mixture into tablets, and
(2-IV) coating the tablets with a coating comprising components (b) and optionally (c) and/or (d).
(2-V) Optionally, the resulting tablets can be film-coated with a suitable film (e).

The preferred processes of the first and second embodiment are described below in more detail.

In step (1-I) or (2-I) components (a), (b), (c) and/or (d) can be provided in micronized form. Micronization can be carried out by milling, such as in a air jet mill. Preferably, the mean particle size (D50) of tasocitinib (a) is from 20 to 120 µm, and from components (b), (c) and/or (d) it is from 30 to 150 µm.

Optionally, the ingredients of the tablet of the invention are blended in order to provide a formulation having a homogenous distribution of tasocitinib (a) within the formulation. Blending can be carried out with conventional mixing devices, e.g. in a free-fall mixer like Turbula^{®} T10B (Bachofen AG, Switzerland). Blending can be carried out e.g. for 1 minute to 30 minutes, preferably for 2 minutes to less than 10 minutes.

Generally, the step (1-II) or (2-II) of "agglomerating" components (a) to (d) (components (c) and (d) optional) refers to a process, wherein particles are attached to each other, thereby giving larger particles. The attachments may occur through physical forces, preferably van der Waals forces. The attachment of particles preferably does not occur through chemical reactions.

Agglomeration (II) can be carried out in different devices. For example, agglomeration can be carried out by a granulation device, preferably by a dry granulation device. More preferably, agglomeration can be carried out by intensive blending. For example, agglomeration can be carried out by blending in a free-fall mixer or a container mixer. An example for a suitable free fall mixer is Turbula^{®} T10B (Bachofen AG, Switzerland). Generally, the blending is carried out for a time, being long enough for agglomeration to occur. Usually, blending is carried out for 10 minutes to 2 hours, preferably for 15 minutes to 60 minutes, more preferably from 20 minutes to 45 minutes.

In a preferred embodiment the agglomeration step is carried out as a dry-compaction step. In a preferred embodiment the dry-compaction step is carried out by roller compaction. Alternatively, e.g. slugging can be used. If roller compaction is applied, the compaction force usually ranges from 1 to 30 kN/cm, preferably from 2 to 20 kN/cm, more preferably from 2 to 10 kN/cm. The gap width of the roller compactor usually is 0.8 to 5 mm, preferably 1 to 4 mm, more preferably 1.5 to 3.2 mm, especially 1.8 to 3.0 mm. After the compaction step, the received comprimate preferably is granulated. Preferably, the granulation step is carried out by an elevated sieving equipment, e.g. Comil^{®} U5 (Quadro Engineering, USA). Alternatively, compaction and granulation can be carried out within one device.

That means, the agglomeration step preferably is carried out in the absence of solvents, preferably in the absence of organic solvents and/or in the absence of water and preferably results in agglomerates or granules.

In a preferred embodiment the agglomeration conditions in step (1-II) or (2-II) are chosen such that the resulting agglomerated pharmaceutical composition comprises a volume mean particle size (D50) of 5 to 500 µm, more preferably of 20 to 250 µm, further more preferably of 50 to 200 µm.

The bulk density of the agglomerated pharmaceutical composition made by the process of the present invention generally ranges from of 0.1 to 0.85 g/ml, preferably of from 0.25 to 0.85 g/ml, more preferably of from 0.3 to 0.75 g/ml.

In a preferred embodiment the composition has a bulk density of 0.5 to 0.8 g/ml when used for direct compressing and 0.1 to 0.5 when used for dry compaction.

The Hausner factor of the agglomerated (or granulated) composition is less than 1.3, preferably less than 1.2 and most preferably less than 1.15. The agglomerated pharmaceutical composition resulting from step (iii) of the invention preferably possesses Hausner ratios in the range of 1.02 to 1.5, preferably of 1.05 to 1.4, more preferably between 1.08 to 1.3. The Hausner ratio is the ratio of tapped density to bulk density. Bulk density and tapped density are determined according to USP 24, Test 616 "Bulk Density and Tapped Density".

The compression step (1-III) or (2-III), can be carried out on a rotary press, e.g. on a Fette^{®} 102i (Fette GmbH, Germany) or a Riva^{®} piccola (Riva, Argentina). If a rotary press is applied, the main compaction force usually ranges from 1 to 50 kN, preferably from 2 to 40 kN, more preferably from 3.5 to 30 kN. The resulting tablets usually have a hardness of 30 to 100N, preferably of 50 to 85 N.

The shell of the tablets of the second preferred embodiment of the present invention is applied in process step (2-IV). Said step comprises coating the tablet core with a coating comprising preferably components (b) and optionally (c) and/or (d). Preferably, the coating comprises components (b), (c) and a plasticizer.

The coating process is generally carried out in a continuously process in a pan coater or a fluid bed dryer. The coating process is preferably carried out on a pan coater, e.g. on a Lödige LHC 25 (Lödige GmbH, Germany). If a pan coater is applied, the spray pressure usually ranges from 0,8 - 2 bar, preferably from 1 - 1.5 bar. The product temperature varies according to the applied polymer. Usually the product temperature is adjusted by 20 - 40 °C, preferably from 32 - 38 °C.

The coating usually has a thickness of 0.01 to 2 mm, preferably from 0.1 to 1.5 mm, more preferably from 0.2 to 1 mm.

After having received the compressed tablets, in both preferred processes the compressed tablet could be film-coated (step 1-IV or 2-V).

In the present invention, the following three types of film-coatings are possible:
e1) film-coating without effecting the release of the active ingredient (preferred),
e2) gastric juice resistant film-coatings,
e3) retard coatings.

Film-coatings without effecting the release of the active ingredient are preferred. Generally, said coating can be water-soluble (preferably having a water solubility at 25 °C of more than 250 mg/ml). With gastric juice resistant coatings, the solubility depends on the pH of the surroundings. Retard coatings are usually non-soluble (preferably having a water solubility at 25 °C of less than 10 mg/ml).

Generally, film-coatings e1) were prepared using cellulose derivatives, poly(meth)-acrylate, polyvinyl pyrrolidone, polyvinyl acetatephthalate, and/or shellac or natural rubbers such as carrageenan.

Preferred examples of coatings, which do not effect the release of the active ingredient, include methylcellulose (MC), hydroxypropyl methylcellulose (HPMC), hydroxypropyl cellulose (HPC), hydroxyethyl cellulose (HEC), polyvinyl pyrrolidone (PVP) and mixtures thereof. These polymers generally have a median molecular weight of 10,000 to 150,000 g/mol.

A preferred polymer is HPMC, most preferably a HPMC having a median molecular weight of 10,000 to 150,000 g/mol and a median level of substitution of -OCH₃-residues of 1.2 to 2.

Examples of gastric juice resistant coatings e2) are cellulose acetate phthalate (CAP), hydroxypropyl methylcellulose phthalate (HPMCP and polyvinyl acetate phthalate (PVAP). Examples of retard coatings e3) are ethyl cellulose (EC, commercially available e.g. as Surelease^{®}) and poly(meth)acrylate (commercially available e.g. as Eudragit^{®} RL or RS and L/S).

The coating e) can be free of active ingredient. However, it is also possible that the coating contains active ingredient (tasocitinib). In such a case, that amount of active ingredient would function as an initial dose. In such a case the coating e) preferably comprises 1 to 45 wt.%, preferably 5 to 35 wt.%, most preferably 10 to 30 wt.% of tasocitinib, based on the total amount of tasocitinib contained in the tablet. In this embodiment, the coating preferably is a coating, which does not effect the release of tasocitinib.

In case the film coating does not contain tasocitinib (which is preferred), it usually has a thickness of 2 µm to 100 µm, preferably from 20 to 60 µm. In case of a coating containing tasocitinib, the thickness of the coating is usually 10 µm to 2 mm, more preferably from 50 to 500 µm.

Accordingly, in a further embodiment the subject invention relates to a tablet in which 1 to 45 wt.%, preferably 5 to 35 wt.%, most preferably 10 to 30 wt.% of the total amount of the tasocitinib contained in the tablet, are present as initial doses having immediate release, and 55 to 99 wt.%, preferably 65 to 95 wt.%, most preferably 70 to 90 wt.% of the active ingredient are present in the tablet as a modified release formulation.

The third preferred embodiment of the present invention relates to a multiple unit pellet system (MUPS). As the name implies, this type of dosage form comprises more than one discrete unit. Typically, such systems comprise 2 to 50, preferably 3 to 30 discrete units. Typically, such discrete units are coated spheroids. Preferably, such coated spheroids are filled into capsules, preferably hard gelatin capsules. Alternatively, such coated spheroids are compressed into tablets.

Hence, a further subject of the present invention is a process for manufacturing an oral modified release dosage form comprising tasocitinib, comprising the steps of
(3-I) providing a pellet core,
(3-II) spraying a solution or suspension comprising component (a) and optionally (d) onto the pellet core,
(3-III) spraying a solution or suspension comprising component (b) and optionally (c) and/or (d) onto the pellet, preferably onto the pellet resulting from step (3-II),
(3-IV) optionally blending the pellets with components (b) and (c) and/or (d); and
(3-V) further processing the resulting mixture into a final oral dosage form.

In this pellet layering embodiment, the present invention provides a process for the manufacture of a modified release dosage form comprising tasocitinib, employing a pellet layering process.

In step (3-I) a pellet core is provided. Preferably, the pellet core is a so-called neutral pellet core, that means it does not comprise an active ingredient. Such pellet cores are known in the art as non-pareils. The pellet core can be made of suitable materials, e.g. cellulose, sucrose, starch or mannitol or combinations thereof.

Suitable pellet cores are commercially available under the trade name Cellets^{®} and preferably comprise a mixture of lactose and microcrystalline cellulose.

Furthermore, in a preferred embodiment, pellet cores commercially available as Suglets^{®} are used. Those preferred pellet cores comprise a mixture of corn starch and sucrose. The mixture usually comprises 1 to 20 wt.% corn starch and 80 to 99 wt.% sucrose, in particular, about 8 wt.% corn starch and 92 % sucrose.

In step (3-II) the tasocitinib is dissolved or suspended in a solvent. The solvent can be water, a pharmaceutically acceptable organic solvent or mixtures thereof. Preferably, the solvent is water or an alcohol. Most preferably, the solvent is methanol.

The solution or dispersion of tasocitinib can comprise further excipients (d). It preferably comprises a solubilizer (d1) and/or a plasticizer (d8). Generally, it is noted that all comments made above regarding the excipients (d) used in the present invention also apply for the processes of the present invention. In addition, the solution or dispersion may comprise anti-sticking agents and lubricants.

The resulting emulsion or suspension is sprayed onto the pellet core, preferably by a fluid bed dryer, e.g. Glatt GPCG 3 (Glatt GmbH, Germany).

Subsequently, the spraying step is repeated. In step (3-III) a solution or suspension comprising component (b) and optionally (c) and/or (d) is sprayed onto the pellet resulting from step (3-II). In the spraying step (3-III), preferably solubilizer (d1) and/or plasticizer (d8) are used as excipients.

Alternatively, the spraying steps (3-II) and (3-III) can be combined. In such a case, the solution or dispersion of tasocitinib further comprises components (b) and optionally (c) and/or excipients (d).

In a preferred embodiment, the spraying conditions are chosen such that the resulting coated spheroids have a mean particle size (D50) of 10 to 1000 µm, more preferably of 50 to 800 µm, further more preferably of 100 to 750 µm, most preferably of 250 to 650 µm.

The coated spheroids of the present invention (i.e. the primary pharmaceutical composition) may be used to prepare suitable solid oral dosage forms with modified released properties. That means, the primary pharmaceutical composition can be further processed to give a "final pharmaceutical composition", i.e. to give a final oral dosage form.

Hence, the present invention encompasses a process for producing oral dosage forms comprising a pharmaceutical composition as received by the above-described pellet layering process, comprising the steps of
(3-V-i) optionally mixing the granulates as received by the above-described pellet layering process with further excipients,
(3-V-ii) further processing the resulting mixture into a final oral dosage form.

Preferably, step (ii) comprises
(3-V-ii-α) filling the resulting mixture into capsules,
(3-V-ii-β) filling the resulting mixture into sachets, or
(3-V-ii-γ) compressing the resulting mixture into tablets. The tablets can be film-coated (e), as described above.

Generally, it is noted that all comments made above with respect to the tablets of the present invention also apply for the process of manufacturing such a tablet and the use of the tablet of the present invention.

Consequently, further subjects of the present invention are tablets obtainable by any of the processes as described above.

All explanations above given for the process of the present invention also apply for the tablet of the present invention.

The release profile of a non-coated tablet or a coated tablet, wherein the coating is free of drug, usually shows a constant release as determined by method USP (paddle). Preferably, the slope of the initial drug release is less than 0.6 to 0.8 % per minute.

In a preferred embodiment, the oral dosage form of the present invention is suitable for administration once or twice per day, most preferably once per day. Alternatively, the oral dosage form of the present invention can be administered every second day, thrice a week, twice a week or once a week.

The present invention also provides the use of the modified release tablet of the present invention as an immunosuppressive agent for organ transplants, xeno transplantation, lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia. The pharmaceutical composition or the oral dosage form of the present invention can be used as an immunosuppressive agent in a method for organ transplants or xenotransplantation, or for treating lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia, said method comprising administering an effective amount of the pharmaceutical composition or the oral dosage form in a subject in need thereof.

The present invention is illustrated by the following examples.

### EXAMPLES

The following commercially available compounds were used in the Examples below:
- Eudragit^{®} L100-55 (Röhm):: anionic copolymer of methacrylic acid and acrylic acid ethylester
- Eudragit^{®} RL PO (Röhm):: copolymer of acrylic and methacrylic acid esters containing quaternary ammonium groups
- Kollicoat^{®} MAE 100P (BASF):: methacrylic acid copolymer
- Kollidon^{®} SR (BASF):: mixture of 80 % hydrophobic polyvinyl acetate, 19 % hydrophilic polyvinyl pyrrolidone, 0.8 % sodium lauryl sulfate and 0.2 % colloidal silicate
- Aerosil^{®} 200 (Degussa):: highly dispersed silicium dioxide
- Avicel^{®} PH102 (FMC):: microcrystalline cellulose, with D50 particle size of about 100 µm
- Lubritab^{®}: hydrogenated vegetable oil
- Opadry^{®}: film-coating

### Examples 1 - 3: Formulations Containing a Pore-Forming Material with pH Dependent Solubility

### Example 1: Matrix tablet, direct compression

**Tablet formulation 1:**

| | |
|---|---|
| Tasocitinib citrate | 10 mg (based on the free base) |
| Eudragit^{®} L100-55 | 40 mg |
| Lactose monohydrate | 30 mg |
| Dicalcium phosphate anhydrate | 30 mg |
| Aerosil^{®} 200 | 1 mg |
| Magnesium stearate | 1 mg |

All ingredients except magnesium stearate were blended in a free fall mixer for 15 min. Then, sieved (500 µm) magnesium stearate was added and the mixture was blended for further 5 min. The final blend was compressed into tablets.

### Example 2: Matrix tablet, wet granulation

**Tablet formulation 2:**

| | |
|---|---|
| Tasocitinib citrate | 10 mg (based on the free base) |
| Kollicoat^{®} MAE 100P | 45 mg |
| Lactose monohydrate | 25 mg |
| Avicel^{®} PH102 | 17 mg |
| Aerosil^{®} 200 | 2 mg |
| Magnesium stearate | 1 mg |

Tasocitinib, Kollicoat^{®} and lactose were sieved (1.25 mm mesh) into the pot of a Diosna^{®} P1-6 wet granulator and blended for 2 min. This pre-mixture was granulated, adding a suitable amount of water to gain a mixture having a "snow ball" consistency. The wet granulate was sieved (2 mm mesh) and dried for 2 h at 40 °C in a cabinet drier. The dried granulate was sieved (1.25 mm mesh) and Avicel^{®} and Aerosil^{®} (both sieved with 1.25 mm mesh) were added and the resulting mixture was blended for further 15 min in a free fall mixer. Sieved (500 µm mesh) magnesium stearate was added and the resulting mixture was blended in a free fall mixer for 5 min. The final blend was compressed into tablets.

### Example 3: Dry granulation

**Tablet formulation 3:**

| | |
|---|---|
| Tasocitinib | 10 mg (based on the free base) |
| Eudragit L 100-55 | 40 mg |
| GalenIQ 800 | 30 mg |
| Dicalciumphosphat anhydrate | 30 mg |
| Aerosil^{®} 200 | 1 mg |
| Magnesium stearate | 1 mg |

All ingredients, except Aerosil 200 and magnesium stearate, were sieved (1 mm mesh) and blended in a free fall mixer for 15 min. The premixture was compacted and the resulting slug was sieved (1mm mesh). Subsequently, Aerosil 200 was added over a sieve (2mm mesh) and blended for further 10 minutes. Then, sieved (500 µm) magnesium stearate was added and the mixture was blended for further 5 min. The final blend was compressed into tablets.

### Examples 4 - 5: Formulations Containing a Pore-Forming Material with pH Independent Solubility

### Example 4: Direct compression

**Tablet formulation 4:**

| | |
|---|---|
| Tasocitinib hemi citrate | 10 mg (based on the free base) |
| Kollidon^{®} SR | 40 mg |
| Lactose monohydrate | 30 mg |
| Dicalcium phosphate anhydrate | 30 mg |
| Aerosil^{®} 200 | 1 mg |
| Magnesium stearate | 1 mg |

All ingredients, except magnesium stearate, were sieved (1 mm mesh) and blended in a free fall mixer for 15 min. Then, sieved (500 µm) magnesium stearate was added and the mixture blended for further 5 min. The final blend was compressed into tablets.

### Example 5: Wet granulation

**Tablet formulation 5:**

| | |
|---|---|
| Tasocitinib citrate | 10 mg (based on the free base) |
| Eudragit^{®} RL PO | 45 mg |
| Lactose monohydrate | 25 mg |
| Avicel^{®} PH102 | 17 mg |
| Aerosil^{®} 200 | 2 mg |
| Magnesium stearate | 1 mg |

Tasocitinib, Eudragit^{®} and lactose were sieved (1.25 mm mesh) into the pot of a Diosna^{®} P1-6 wet granulator and blended for 2 min. This pre-mixture was granulated, adding a suitable amount of water to gain a mixture having a "snow ball" consistency. The wet granulate was sieved (2 mm mesh) and dried for 2 h at 40 °C in a cabinet drier. The dried granulate was sieved (1.25 mm mesh) and Avicel^{®} and Aerosil^{®} (both sieved with 1.25 mm mesh) were added and the resulting mixture was blended for further 15 min in a free fall mixer. Sieved (500 µm mesh) magnesium stearate was added and the resulting mixture was blended in a free fall mixer for 5 min. The final blend was compressed into tablets.

### Example 6: Coated tablet

**Tablet formulation 6:**

| Tablet core | |
|---|---|
| Tasocitinib | 10 mg (based on the free base) |
| StarLac^{®} | 80 mg |
| Dicalciumphosphat anhydrate | 10 mg |
| Aerosil 200 | 1 mg |
| Magnesiumstearate | 1 mg |

All excipients, excluding magnesium stearate, were sieved (800µm) and mixed together for 15 min in a free fall mixer. Sieved (500 µm mesh) magnesium stearate was added and the resulting mixture was blended in a free fall mixer for 5 min. The final blend was compressed into tablets.

**Tablet coating**

| | |
|---|---|
| Ethylcellulose | 20 mg |
| PEG 6000 | 1 mg |
| TEC | 5 mg |

The coating process was carried out on a pan coater, e.g. on a Lödige LHC 25 (Lödige GmbH, Germany). The spray pressure usually ranges from 1 - 1.5 bar. The product temperature varies according to the applied polymer from 32 - 38 °C.

### Example 7: MUPS

**Tablet formulation 7:**

| | |
|---|---|
| Tasocitinib, micronized | 10 mg |
| Polyoxyethylenepropylene copolymer | 4 mg |
| Ethylcellulose: | 15 mg |
| PEG 4000 | 4 mg |
| Nonpareils | 40 mg |
| MCC | 200 mg |
| Polyvinylpyrrolidone | 10 mg |
| Lubritab^{®} | 5 mg |
| Aerosil^{®} | 2 mg |
| Opadry^{®} | 2.5 mg |

### Procedure:

Tasocitinib was suspended together with ethyl cellulose in an aqueous solution of polyoxyethylene propylene copolymer and PEG. The placebo pellets were pre-heated to 38 °C in a fluid bed dryer. Subsequently the pellets were coated with the suspension, using the following parameter:

| | |
|---|---|
| Inlet temperature: | 40-80°C |
| Product temperature: | 35-40°C |
| Spray nozzle: | 1 - 2 mm |
| Spray pressure: | 1 - 2 bar |

After sintering at elevated temperature the pellets were blended with MCC and Aerosil^{®} and polyvinylpyrrolidone for 25 min in a tumble blender. Afterwards, Lubritab^{®} was added and the blend was mixed for additional 3 minutes.

The final blend was compressed on a Fette^{®} 102 rotary press, characterized by following parameters:

| | |
|---|---|
| Hardness: | 80 -110 N |
| Friability: | less than 1 %. |

The tablets were film-coated in order to achieve a better compliance with an aqueous solution of Opadry^{®} (Colorcon^{®}):

| | |
|---|---|
| Product temperature: | 37 - 40°C |
| Supply air temperature: | 40 - 80°C |
| Nozzle diameter: | 1,2 mm |
| Spray pressure: | 1 -3 bar |

Afterwards, the tablets were sintered at 60 °C for 0.5 hour.

## Claims

1. Oral dosage form for modified release comprising
(a) tasocitinib, and
(b) a non-erodible material.

2. Oral dosage form according to claim 1, wherein. tasocitinib is contained in an amount of 1 to 60 wt.%, based upon the total weight of the oral dosage form.

3. Oral dosage form according to claim 1 or 2, wherein the non-erodible material has a solubility in water at 25 °C at a pH of 5.0 of less than 33 g/l.

4. Oral dosage form according to anyone of the previous claims, wherein the non-erodible material has a solubility in water at 25 °C at a pH of 7.0 of more than 33 g/l.

5. Oral dosage form according to anyone of the previous claims, wherein the non-erodible material is a non-erodible polymer, preferably having a weight average molecular weight from 30,000 to 3,000,000 g/mol.

6. Oral dosage form according to anyone of the previous claims, wherein the non-erodible material is contained in an amount of 5 to 80 wt.%, based upon the total weight of the oral dosage form.

7. Oral dosage form according to any of the previous claims, further comprising a pore-forming material (c).

8. Oral dosage form according to claim 7, wherein the pore-forming material has a solubility in water at 25 °C and at a pH of 5.0 of more than 50 g/l.

9. Oral dosage form according claims 7 or 8, wherein the pore-forming material is contained in an amount of 1 to 50 wt.%, preferably from 5 to 40 wt.%, based upon the total weight of the oral dosage form.

10. Oral dosage form according to anyone of the previous claims, further comprising at least one further excipient (d) selected from solubilizers, fillers, lubricants, disintegrants, glidants, anti-sticking agents, plasticizers and mixtures thereof.

11. Oral dosage form according to anyone of the previous claims in form of a matrix tablet.

12. Oral dosage form according to anyone of claims 1 to 10 in form of a tablet comprising a core and a shell, wherein the core comprises components (a) and optionally (c) and/or (d) and wherein the shell comprises components (b) and optionally (c) and/or (d).

13. Oral dosage form according to anyone of claims 1 to 10 in form of a multiple unit pellet system.

14. Process for manufacturing a tablet according to anyone of claims 1 to 11 comprising the steps of
(1-I) providing components (a), (b), optionally (c), and optionally (d),
(1-II) optionally agglomerating the components of step (I) to yield granules,
(1-III) compressing the mixture resulting from step (I) or (II) into tablets; and
(1-IV) optionally film-coating the tablets.

15. Process for manufacturing a tablet according to anyone of claims 1 to 10 or 12 comprising the steps of
(2-I) mixing components (a) and optionally (c) and/or (d),
(2-II) optionally agglomerating the components of step (I) to yield granules,
(2-III) compressing the mixture into tablets, and
(2-IV) coating the tablets with a coating comprising components (b) and optionally (c) ad/or (d).

16. Process for manufacturing an oral dosage form according to anyone of claims 1 to 10 or 13 comprising the steps of
(3-I) providing a pellet core,
(3-II) spraying a solution or suspension comprising component (a) and optionally (d) onto the pellet core,
(3-III) spraying a solution or suspension comprising component (b) and optionally (c) and/or (d) onto the pellet, preferably onto the pellet resulting from step (3-II),
(3-IV) optionally blending the pellets with components (b) and (c) and/or (d); and
(3-V) further processing the resulting mixture into a final oral dosage form.
